# EUROPEAN PATENT APPLICATION

(11) **EP 4 134 449 A1**
(43) Date of publication of application: **15.02.2023**
(21) Application number: 21194909.4
(22) Date of filing: 03.09.2021
(51) Int. Cl.: C12Q 1/6883

(54) **METHOD FOR SHEEP SELECTION BASED ON PARASITE RESISTANT GENOTYPES**

(30) Priority: 13.08.2021 PT 2021117398
(71) Applicant: ACOS - Associação de Agricultores do Sul, 7800-453 Beja (PT); Cebal - Centro De Biotecnologia Agrícola E Agro-Alimentar Do Alentejo, 7800-309 Beja (PT); Universidade De Évora, 7004-516 Évora (PT); INIAV - Instituto Nacional de Investigação Agrária E Veterinária, 2780-157 Oeiras (PT)
(72) Inventor: PEREIRA MATOS, Claudino António, 7800-453 BEJA (PT); COSTA DO AMARAL RAMOS, António Marcos, 7800-309 BEJA (PT); USIÉ CHIMENOS, Ana Isabel, 7800-309 BEJA (PT); BRANCO GASPAR, Daniel Filipe, 7800-309 BEJA (PT); GODINHO VIEIRA MONTEIRO, Maria Helena, 7800-453 BEJA (PT); FIALHO LEÃO, Célia Cristina, 2780-157 OEIRAS (PT); PIMENTEL CAROLINO, Renato Nuno, 2005-048 VALE DE SANTARÉM (PT); DA SILVA BRANCO, Sandra Maria, 7002-554 ÉVORA (PT); NETO PADRE, Ludovina, 7002-554 ÉVORA (PT); VARELA BETTENCOURT HENRIQUES, Elisa Maria, 7002-554 ÉVORA (PT); DE SOUSA HENRIQUES, Pedro Damião, 7000-803 ÉVORA (PT)
(74) Representative: Neves, Ana

(57) **Abstract**

The present patent application discloses a method for the selection of sheep based on their parasite resistance profile. Said parasite resistance profile is determined based on a group of Single Nucleotide Polymorphisms (SNPs) herein associated with lower scores of gastrointestinal parasite eggs (GPE) in sheep.

## Description

### Technical field

The present patent application relates to a method tor the selection of sheep based on parasite resistant genotypes.

### Background art

In sheep, gastrointestinal parasites represent a significant problem. The eggs of these parasites are found on pasture and are then ingested by the sheep during their typical grazing behavior. These parasites are associated with a variety of symptoms in sheep that include weight loss, diarrhea, weakness, and anemia, which in turn are associated with severe production losses. Treatment for these parasites involves the use of various drugs, called anthelmintics, which are administered to infected sheep. However, these treatments represent an additional cost for producers, and there are also reports of resistance to the anthelmintic drugs used in sheep and other ruminants. Therefore, genetic selection of animals with greater resistance to gastrointestinal parasites can be a very efficient tool in combating this problem.

In sheep, there is variation in the individual response of each animal to gastrointestinal parasites. Under the same environmental conditions, for example, on the same farm, some animals are severely affected by the disease, while others show no symptoms and do not seem to be affected. This situation clearly indicates the possibility of genetic control of gastrointestinal parasites and, in particular, the severity with which each animal is affected. To date, no genetic markers for gastrointestinal parasites have been identified in Portuguese sheep breeds.

### Summary

The present patent application relates to a method for the selection of sheep based on parasite resistant genotypes.

The method for sheep selection based on parasite resistant genotype comprises the following steps:
Preparation of a sheep biological sample and extraction of DNA from said sample;
Genotyping the DNA sample for at least one of the following Single Nucleotide Polymorphisms (SNPs),

| Chromosome | Position | Exonic alteration |
|---|---|---|
| NC_040256.1 | 20660687 | - |
| NC_040263.1 | 45551043 | synonym |
| NC_040264.1 | 80485067 | - |
| NC_040268.1 | 65106946 | - |
| NC_040272.1 | 26492975 | - |
| NC_040252.1 | 189900946 | - |
| NC_040258.1 | 21158733 | - |
| NC_040259.1 | 65157854 | - |
| NC_040267.1 | 61398933 | - |
| NC_040274.1 | 32118726 | - |
| NC_040278.1 | 102779687 | - |

Determining sheep's resistance to parasites based on the presence of at least one of said SNPs significantly associated with a lower score for the amount of gastrointestinal parasite eggs.

In one embodiment, the sample analyzed is preferably a blood sample.

In another embodiment, the above-mentioned Single Nucleotide Polymorphyms (SNPs) are used as biomarkers of parasite resistance in sheep.

### Detailed Description

The present patent application discloses a method for the selection of sheep based on parasite resistant profile. Said parasite resistance profile is determined based on a group of Single Nucleotide Polymorphisms (SNPs) disclosed herein which are associated with lower scores of gastrointestinal parasite eggs (GPE) in sheep.

The technology disclosed herein is based on the sequencing of genomes of animals of the breeds that are subsequently used in genome-wide association studies. This step of genome sequencing is essential to characterize in great detail all the type of variation present in the animals studied. The following step involved selecting a set of SNPs to be genotyped in a larger number of animals, which in this case was 1500. All genotyped animals were also characterized from a phenotypic point of view, namely in determining the amount of gastrointestinal parasite eggs (GPE) observed in each animal.

After all animals were genotyped, a genome-wide association study was performed, where the statistical significance of the association observed between each of the genotyped SNPs and the phenotypic value of the amount of GPEs was determined. Since the distribution of phenotypic data did not conform to the characteristics of a normal distribution, a logarithmic transformation of the data was performed before the genome-wide association analysis. The significance of each SNP was determined after a correction for multiple testing, an essential step in this type of analysis.

For each of the significant SNPs the observed mean for each of the genotypes is determined. The difference observed is the potential phenotypic gain that can be achieved if animals carrying the genotypes associated with lower OPG score are genetically selected.

The 11 SNPs identified are described in Table 1.

| Chromosome | Position | P-Value | Gene ID | Genomic Localization | Exonic alteration |
|---|---|---|---|---|---|
| NC_040256.1 | 20660687 | 0.04863 | gene-PDLIM4 | UTR3 | - |
| NC_040263.1 | 45551043 | 0.04016 | gene-DISP3 | exon | synonym |
| NC_040264.1 | 80485067 | 0.04016 | gene-ARFGEF2 | intron | - |
| NC_040268.1 | 65106946 | 0.04863 | gene-HSPB8 | intron | - |
| NC_040272.1 | 26492975 | 0.03608 | gene-IGSF22 | UTR3 | - |
| NC_040252.1 | 189900946 | 0.04016 | | intergenic | - |
| NC_040258.1 | 21158733 | 0.03608 | | intergenic | - |
| NC_040259.1 | 65157854 | 0.04701 | | intergenic | - |
| NC_040267.1 | 61398933 | 0.04016 | | intergenic | - |
| NC_040274.1 | 32118726 | 0.04016 | | intergenic | - |
| NC_040278.1 | 102779687 | 0.04086 | | intergenic | - |

For the 11 SNPs identified, the observed mean values are shown in Table 2.

| NC_040256.1 | NC_040256.1#20660687 | GENO | T/T | T/C | C/C |
|---|---|---|---|---|---|
| NC_040256.1 | NC_040256.1#20660687 | COUNTS | 171 | 544 | 661 |
| NC_040256.1 | NC_040256.1#20660687 | FREQ. | 0.1243 | 0.3953 | 0.4804 |
| NC_040256.1 | NC_040256.1#20660687 | MEAN | 0.5205 | 0.8658 | 0.9259 |
| NC_040256.1 | NC_040256.1#20660687 | SD | 0.8768 | 0.9741 | 0.9734 |
| | | | | | |
| NC_040263.1 | NC_040263.1#45551043 | GENO | C/C | C/T | T/T |
| NC_040263.1 | NC_040263.1#45551043 | COUNTS | 283 | 589 | 488 |
| NC_040263.1 | NC_040263.1#45551043 | FREQ | 0.2081 | 0.4331 | 0.3588 |
| NC_040263.1 | NC_040263.1#45551043 | MEAN | 1.042 | 0.8812 | 0.7254 |
| NC_040263.1 | NC_040263.1#45551043 | SD | 0.9812 | 0.9704 | 0.954 |
| | | | | | |
| NC_040264.1 | NC_040264.1#80485067 | GENO | T/T | T/C | C/C |
| NC_040264.1 | NC_040264.1#80485067 | COUNTS | 68 | 481 | 843 |
| NC_040264.1 | NC_040264.1#80485067 | FREQ | 0.04885 | 0.3455 | 0.6056 |
| NC_040264.1 | NC_040264.1#80485067 | MEAN | 0.5441 | 0.7547 | 0.9454 |
| NC_040264.1 | NC_040264.1#80485067 | SD | 0.8183 | 0.9906 | 0.9695 |
| | | | | | |
| NC_040268.1 | NC_040268.1#65106946 | GENO | G/G | G/A | A/A |
| NC_040268.1 | NC_040268.1#65106946 | COUNTS | 133 | 549 | 703 |
| NC_040268.1 | NC_040268.1#65106946 | FREQ | 0.09603 | 0.3964 | 0.5076 |
| NC_040268.1 | NC_040268.1#65106946 | MEAN | 1.075 | 0.9271 | 0.7511 |
| NC_040268.1 | NC_040268.1#65106946 | SD | 1.005 | 0.99 | 0.9333 |
| | | | | | |
| NC_040272.1 | NC_040272.1#26492975 | GENO | C/C | C/T | T/T |
| NC_040272.1 | NC_040272.1#26492975 | COUNTS | 33 | 263 | 1094 |
| NC_040272.1 | NC_040272.1#26492975 | FREQ | 0.02374 | 0.1892 | 0.7871 |
| NC_040272.1 | NC_040272.1#26492975 | MEAN | 1.121 | 1.095 | 0.7888 |
| NC_040272.1 | NC_040272.1#26492975 | SD | 1.053 | 0.9858 | 0.9585 |
| | | | | | |
| NC_040252.1 | NC_040252.1#189900946 | GENO | T/T | T/C | C/C |
| NC_040252.1 | NC_040252.1#189900946 | COUNTS | 22 | 236 | 1145 |
| NC_040252.1 | NC_040252.1#189900946 | FREQ | 0.01568 | 0.1682 | 0.8161 |
| NC_040252.1 | NC_040252.1#189900946 | MEAN | 0.4091 | 0.6356 | 0.9048 |
| NC_040252.1 | NC_040252.1#189900946 | SD | 0.7964 | 0.9241 | 0.9773 |
| | | | | | |
| NC_040258.1 | NC_040258.1#21158733 | GENO | C/C | C/T | T/T |
| NC_040258.1 | NC_040258.1#21158733 | COUNTS | 12 | 244 | 1160 |
| NC_040258.1 | NC_040258.1#21158733 | FREQ | 0.008475 | 0.1723 | 0.8192 |
| NC_040258.1 | NC_040258.1#21158733 | MEAN | 1.417 | 1.094 | 0.7991 |
| NC_040258.1 | NC_040258.1#21158733 | SD | 0.793 | 0.9704 | 0.9663 |
| | | | | | |
| NC_040259.1 | NC_040259.1#65157854 | GENO | C/C | C/T | T/T |
| NC_040259.1 | NC_040259.1#65157854 | COUNTS | 40 | 400 | 953 |
| NC_040259.1 | NC_040259.1#65157854 | FREQ | 0.02872 | 0.2872 | 0.6841 |
| NC_040259.1 | NC_040259.1#65157854 | MEAN | 1.175 | 1 | 0.7817 |
| NC_040259.1 | NC_040259.1#65157854 | SD | 0.9306 | 1.006 | 0.9585 |
| | | | | | |
| NC_040267.1 | NC_040267.1#61398933 | GENO | C/C | C/T | T/T |
| NC_040267.1 | WC_040267.1#61398933 | COUNTS | 181 | 638 | 584 |
| NC_040267.1 | NC_040267.1#61398933 | FREQ | 0.129 | 0.4547 | 0.4163 |
| NC_040267.1 | NC_040267.1#61398933 | MEAN | 0.6409 | 0.7978 | 0.9726 |
| NC_040267.1 | NC_040267.1#61398933 | SD | 0.9179 | 0.9631 | 0.9867 |
| | | | | | |
| NC_040274.1 | NC_040274.1#32118726 | GENO | C/C | C/T | T/T |
| NC_040274.1 | NC_040274.1#32118726 | COUNTS | 100 | 516 | 746 |
| NC_040274.1 | NC_040274.1#32118726 | FREQ | 0.07342 | 0.3789 | 0.5477 |
| NC_040274.1 | NC_040274.1#32118726 | MEAN | 1.07 | 0.9671 | 0.7507 |
| NC_040274.1 | NC_040274.1#32118726 | SD | 0.9129 | 0.9679 | 0.976 |
| | | | | | |
| NC_040278.1 | NC_040278.1#102779687 | GENO | C/C | C/G | G/G |
| NC_040278.1 | NC_040278.1#102779687 | COUNTS | 12 | 196 | 1198 |
| NC_040278.1 | NC_040278.1#102779687 | FREQ | 0.008535 | 0.1394 | 0.8521 |
| NC_040278.1 | NC_040278.1#102779687 | MEAN | 1.25 | 1.117 | 0.8063 |
| NC_040278.1 | NC_040278.1#102779687 | SD | 0.866 | 0.9179 | 0.9764 |

The method of the present patent application involves several steps. Initially, it is necessary to collect a sample of biological material, such as a blood sample, for DNA extraction. Next, this DNA sample is genotyped for the SNPs of interest, and the genotypes obtained for each SNP are evaluated. The decision to select the animal is based on the presence of the genotype(s) significantly associated with a lower score for the amount of GPEs, i.e. the goal is to select animals with higher resistance to gastrointestinal parasites.

The method for sheep selection based on parasite resistant genotype comprises the following steps:
Preparation of a sheep biological sample and extraction of DNA from said sample;
Genotyping the DNA sample for at least one of the following Single Nucleotide Polymorphisms (SNPs),

| Chromosome | Position | Exonic alteration |
|---|---|---|
| NC_040256.1 | 20660687 | - |
| NC_040263.1 | 45551043 | synonym |
| NC_040264.1 | 80485067 | - |
| NC_040268.1 | 65106946 | - |
| NC_040272.1 | 26492975 | - |
| NC_040252.1 | 189900946 | - |
| NC_040258.1 | 21158733 | - |
| NC_040259.1 | 65157854 | - |
| NC_040267.1 | 61398933 | - |
| NC_040274.1 | 32118726 | - |
| NC_040278.1 | 102779687 | - |

Determining sheep's resistance to parasites based on the presence of at least one of said SNPs significantly associated with a lower score for the amount of gastrointestinal parasite eggs.

In one embodiment, the sample analyzed is preferably a blood sample.

## Claims

1. Method for sheep selection based on parasite resistant genotypes comprises the following steps:
Preparation of a sheep biological sample and extraction of DNA from said sample;
Genotyping the DNA sample for at least one of the following Single Nucleotide Polymorphisms (SNPs),
| Chromosome | Position | Exonic alteration |
|---|---|---|
| NC_040256.1 | 20660687 | - |
| NC_040263.1 | 45551043 | synonym |
| NC_040264.1 | 80485067 | - |
| NC_040268.1 | 65106946 | - |
| NC_040272.1 | 26492975 | - |
| NC_040252.1 | 189900946 | - |
| NC_040258.1 | 21158733 | - |
| NC_040259.1 | 65157854 | - |
| NC_040267.1 | 61398933 | - |
| NC_040274.1 | 32118726 | - |
| NC_040278.1 | 102779687 | - |
Determining sheep's resistance to parasites based on the presence of the at least one of said SNPs significantly associated with a lower score for the amount of gastrointestinal parasite eggs.

2. Single Nucleotide Polymorphyms (SNPs) as defined in claim 1 for use as biomarkers of parasite resistance in sheep.
